Europäisches Patentamt

European Patent Office

Office européen des brevets

(11) Publication number: 0 357 856
A1

# EUROPEAN PATENT APPLICATION

(21) Application number: 88312271.5

(22) Date of filing: 23.12.88

(51) Int. Cl.5· **C12N 15/00 , C12P 21/02 , C07K 15/00 , C12P 21/00 , A61K 39/395 , G01N 33/68 , //(C12P21/00,C12R1:91)**

The microorganism(s) has (have) been deposited with the American Type Culture Collection under number(s) ATCC 67789 and 67791.

(30) Priority: 08.09.88 US 241672

(43) Date of publication of application:
**14.03.90 Bulletin 90/11**

(84) Designated Contracting States:
**AT BE CH DE ES FR GB GR IT LI LU NL SE**

(71) Applicant: **Sansone, Antonio**
**111 Petermar**
**Woodbridge Ontario L4L 1A6(CA)**

(72) Inventor: **Jackowski, George**
**1263 Kipling Avenue**
**Islington, Ontario M9B 3N4(CA)**

(74) Representative: **Paget, Hugh Charles Edward**
**et al**
**MEWBURN ELLIS & CO. 2/3 Cursitor Street**
**London EC4A 1BQ(GB)**

(54) DNA coding for human ventricular myosin light chain 2.

(57) The DNA segment is provided having a sequence which codes for human ventricular myosin light chain 2 (HVMLC2) protein, or a fragment of the DNA segment. The DNA segment or a fragment of the DNA segment of the invention can be used to produce HVMLC2 protein or a part of the protein by culturing a transformant microorganism which includes a recombinant plasmid comprising a plasmid vector into which the DNA segment or a fragment of the DNA segment of the invention has been inserted.

EP 0 357 856 A1

## DNA CODING FOR HUMAN VENTRICULAR MYOSIN LIGHT CHAIN 2

The invention relates to a DNA segment having a sequence which codes for human ventricular myosin light chain 2 (HVMLC2) protein or a fragment of the DNA segment. The DNA segment of the invention can be used to produce human ventricular myosin light chain 2 protein or part of the protein by culturing, a transformant microorganism which includes a recombinant plasmid comprising a plasmid vector into which the DNA segment of the invention or a fragment of the DNA segment has been inserted. The DNA segment of the invention also permits selection of oligonucleotide fragments and polypeptides which are unique to human ventricular myosin light chain 2 protein or to myosin light chain proteins from other species and/or tissues.

Myosin is the major protein constituent of cardiac muscle and it consists of two myosin heavy chains and four myosin light chains. Two classes of light chains have been characterized in cardiac myosin: cardiac myosin light chain 1 with a molecular weight of 27,000 daltons and myosin light chain 2 with a molecular weight of 20,000 daltons.

The light chain of myosin is released from cardiac muscle following myocardial infarction and may remain elevated in serum for several days (Khaw et al, Circulation 58 p.1130, 1978 and Trahern et al. Am. J. Cardiol 41 p. 641, 1978). Katus et al. (JACC, Vol. 2 (3) p. 487) recently reported a correlation between the presence of myosin light chains and signs of ischemia in the electrocardiogram and extent of coronary artery narrowing. An elevated plasma light chain concentration has also been observed in patients with congestive cardiomyopathy associated with an inflammatory infiltrate (Haver, E., J. Mol. & Cell Cardiol. 17 (Supplement 2) p.53, 1985).

There are a number of reports describing diagnostic methods for muscle diseases utilizing polyclonal or monoclonal antibodies directed at myosin light chains (Trahern et al. supra; Khaw et al. Supra; Nagai et al. Biochem. Biophys. Res. Commun. 86 p.683, 1979; and Katus et al. Am. J. Cardiol. 54 p.964, 1984, Katus et al. Mol. Immuno. 19 p.451, 1982, and Masahito et al. European Patent Application D205 177 A2). The diagnostic methods however suffer from a number of disadvantages such as cross-reactivity between cardiac myosin light chains and myosin light chains from other tissues or species.

Polyclonal or monoclonal antibodies specific for cardiac myosin light chains can be used for various types of therapy. For example, such antibodies can be conjugated to a radioisotope to image damaged cardiac muscle tissue or to a therapeutic agent to deliver the agent to damaged cardiac muscle tissue.

The identification of the DNA sequences that code for human myosin light chains would facilitate the preparation of highly specific monclonal and polyclonal antibodies which would be useful in diagnostic methods and for various types of therapy.

Weeds and Pope (Nature, 234, p. 85. 1981) examined thiol sequences of cardiac light chains from bovine and sheep hearts. The amino acid sequence of myosin light chains from normal and hypertrophied human hearts was partially elucidated by Klotz et al. (Circulation Research 50, p. 201, 1982). Klotz et al. sequenced peptides isolated from human cardiac myosin light chain 2 and found that their sequences corresponded to 70% of the whole molecule. Collins et al. (Bioscience Reports 6, p.655, 1986) disclosed the amino acid sequence for rabbit ventricular myosin light chain 2 (LC2).

A few studies have been directed at characterizing the nucleotide sequences of cardiac myosin light chains. The present inventor has described a DNA segment having a sequence which codes for human ventricular myosin light chain 1 protein (U.S .Patent Application Serial No. 07/160,892 filed February 26, 1988). Kumar et al. (J. Biol. Chem. 261 p.2866, 1986) has described the nucleotide sequence of a recombinant plasmid containing cDNA corresponding to rat heart myosin light chain 2.

The present inventor using a cDNA cloning method has identified and sequenced a DNA segment having a sequence which codes for the HVMLC2 protein. The HVMLC2 protein has hitherto not been completely sequenced at the protein level using conventional methods due to the blockage of the N-terminal end of the protein. In addition, at the protein level HVMLC2 protein is readily degradable. The HVMLC2 protein has also hitherto not been sequenced by DNA cloning methods due to difficulties in isolating mRNA from cardiac tissue since it is readily degraded by RNase, and difficulties in obtaining sufficient quantities of human material for characterization. Furthermore, the mRNA that codes for HVMLC2 is presnt in extremely small quantities and it is contaminated with much larger amounts of other mRNA's that act similarly to it in the cloning methodology and which are not related to HVMLC2. Therefore, at each stage of the cloning method, a complex mixture is encountered, only a very small part of which is related to the HVMLC2 protein.

The present invention therefore provides a means and method of screening complex mixtures of mRNA in order to select the mRNA that codes for HVMLC2 protein. The present invention further provides a DNA segment having a sequence which codes for HVMLC2 protein, or a fragment of the DNA segment.

In accordance with one embodiment of the invention, the DNA segment has a sequence which codes for HVMLC2 protein of the amino acid sequence as shown in Table I. In accordance with a second embodiment of the invention the DNA segment has a sequence which codes for HVMLC2 protein having the nucleotide base sequence as shown in Table I. In accordance with a third embodiment of the invention the DNA segment of the invention has the nucleotide sequence as shown in Table II.

The invention also contemplates a DNA segment or a fragment of the DNA segment hybridizing to a DNA segment or a fragment of DNA segment of the invention from whatever source obtained including natural, synthetic or semisynthetic sources, that is related by mutations, including nucleotide substitutions, nucleotide deletions, nucleotide insertions and inversions of nucleotide stretches to the DNA segment or arrangment of the DNA segment according to the invention and having a sequence which codes for HVMLC2 protein.

The invention further contemplates a recombinant plasmid adapted for transformation of a host comprising a plasmid vector into which a DNA segment having a sequence which codes for HVMLC2 protein, or a fragment of the DNA segment, has been inserted. Preferred recombinant plasmids are those comprising a plasmid vector into which a DNA segment having a sequence which codes for a HVMLC2 protein of the amino acid sequence as shown in Table I, or of the nucleotide sequence as shown in Table I, or a fragment of such a DNA segment, has been inserted. The recombinant plasmid of the invention can be introduced into a host cell which can then be cultured to produce HVMLC2 protein or a part of the protein.

The invention also provides a process for producing HVMLC2 protein or a part of the protein comprising culturing a transformant host cell including a recombinant plasmid comprising a plasmid vector into which a DNA segment having a sequence which codes for HVMLC2 protein or a fragment of the DNA segment has been inserted, in a suitable medium until HVMLC2 protein or a part of the protein is formed and thereafter isolating the HVMLC2 protein or a part of the protein.

The invention further contemplates a polyclonal or monoclonal antibody directed at HVMLC2 protein or a part of the protein prepared in accordance with the methods of the invention, methods for preparing the polyclonal or monoclonal antibody and diagnostic methods for cardiac muscle diseases using the polyclonal or monoclonal antibody. The invention further contemplates a medicament for the prophylaxis and treatment of heart disease which comprises a polyclonal or monoclonal antibody directed at HVMLC2 protein prepared in accordance with the methods of the invention and one or more of a therapeutic agent and a pharmaceutically acceptable excipient, diluent and auxiliary. The invention still further contemplates an agent for detecting damaged cardiac tissue which comprises a polyclonal or monoclonal antibody directed at HVMLC2 protein or part of the protein prepared in accordance with the methods of the invention and a labelling agent.

The present invention permits, for the first time, the precise identification of a polypeptide sequence or a part of a polypeptide sequence, or a nucleotide sequence or a part of a nucleotide sequence, which is unique to HVMLC2. To those skilled in the art, such polypeptide or nucleotide sequences of HVMLC2, with their detailed conformations deduced on energetic and thermodynamic groups, enable the separation with unprecedented specificity of HVMLC2 by immunologic (e.g. based on poly- or monoclonal antibody against a polypeptide or a part of a polypeptide, used alone or in concert), by nucleotide hybridization techniques (e.g. using DNA or RNA complementary strands with/without signal amplifications), by receptor-based or receptor-like assays, or by other variants of these well-known methods.

## DESCRIPTION OF THE DRAWINGS

The invention will be better understood with reference to the drawings in which:

Figure 1 is a schematic illustration of a restriction map of a plasmid containing the DNA segment of one embodiment of the invention.

Figure 2 shows the conformation of HVMLC2 constructed from the deduced amino acid sequence of the DNA segment according to one embodiment of the invention.

## DETAILED DESCRIPTION

In accordance with one embodiment of the invention, the HVMLC2 protein encoded in the DNA segment of the invention is composed of the 165 amino acids shown in Table I. A comparison of the deduced amino acid sequence of the HVMLC2 protein encoded in the DNA segment of the embodiment of the invention shown in Table I with the known partial amino acid sequence determined from chymotryptic peptides of human cardiac myosin light chain 2 by Klotz et al, (Circulation Research, 50, p. 201, 1982) indicates that the amino acid sequences are similar (Table III). The deduced amino acid sequence of the HVMLC2 protein encoded in the DNA of the embodiment of the invention is also similar to the amino acid sequence of rabbit cardiac light chain LC2 (Collins et al, supra) and the deduced amino acid sequence of rat cardiac light chain LC2 (Kumar et al, supra) (Table IV). The similarities between the rabbit and rat and the human cardiac myosin light chain sequences indicate that the rabbit and rat sequences as well as antibodies directed against the rabbit and rat light chain 2 proteins, may be useful as standards in diagnostic methods for detecting HVMLC2 protein or DNA or mRNA sequences of HVMLC2.

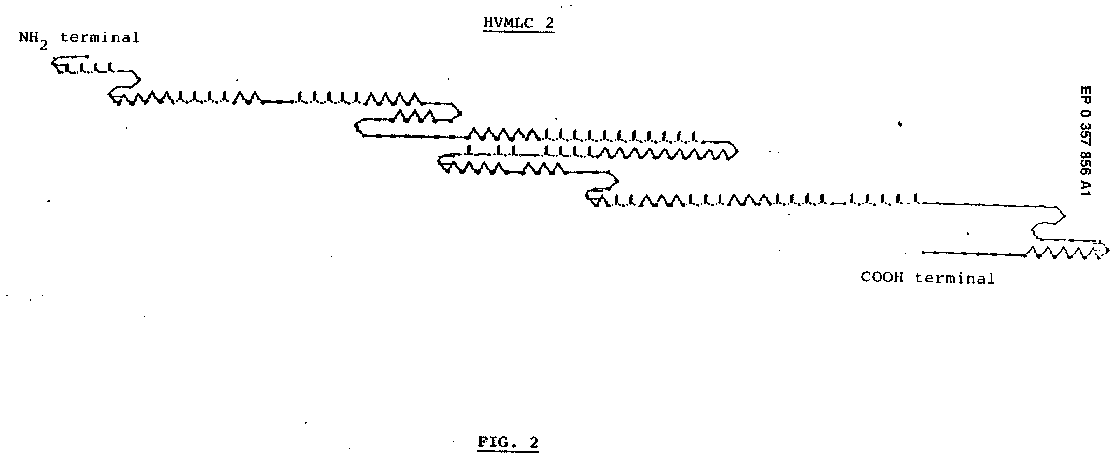

The conformation of a protein may be deduced only after the complete amino acid sequence of the protein is known. Accordingly, the confirmation of the HVMLC2 protein may be deduced from the amino acid sequence identified in the embodiment of the invention. Figure 2 shows the conformation of the HVMLC2 protein deduced from the amino acid sequence shown in Table I. As shown in Figure 2, α-helical structures are found at the proline residues of the amino acid sequence shown in Table I and there are polypeptides located at exposed sites in the HVMLC2 protein.

A comparison of the amino acid sequence of HVMLC2 protein identified in the embodiment of the present invention with the amino acid sequences of myosin light chain proteins described in the literature, as well as the conformations of these proteins, indicates that there are sequences which are unique to HVMLC2. It is the unique sequences of the HVMLC2 protein encoded in the DNA segment of the present invention and the specific conformation of the protein which permits specific differentiation of ventricular myosin light chain 2 protein from myosin light chain proteins from other species and/or tissues.

A comparison of the deduced amino acid sequence identified in the embodiment of the present invention with the deduced amino acid sequence of rat cardiac light chain LC2 (Kumar et al, supra), indicates that the following polypeptides are unique to HVMLC2.

Ala-Gly-Gly-Ala-Asn (amino acids 10 to 14 in Table I)
Asp-Pro-Glu-Gly-Lys-Gly-Val-Leu-Lys-Ala-Asp-Tyr-Val-Arg (amino acids 106 to 119 in Table I)
The polypeptides are located at exposed sites and are particularly useful in differentiating HVMLC2. (Figure 2). The polypeptides unique to HVMLC2 can be synthetically synthesized using solid phase synthesis (Eberle, A.N.J. Chem. Soc. Perkin. Trans, 1, p.361, 1986).

In the nucleotide base sequence shown in Table II, the nucleotide positions from 1 to 102 represent the oligo dG tailed linker used in the cloning, and those from 103 to 146 represent the noncoding area. The ATG sequence at positions 147 to 149 is the start codon. The sequence between positions 150 and 647 is the portion that encodes the HVMLC2 protein. The sequence at positions 648 to 650 is the stop codon and the sequence after position 650 is the 3′-noncoding region.

The DNA segment of the present invention can be prepared, for example, by the method as described below.

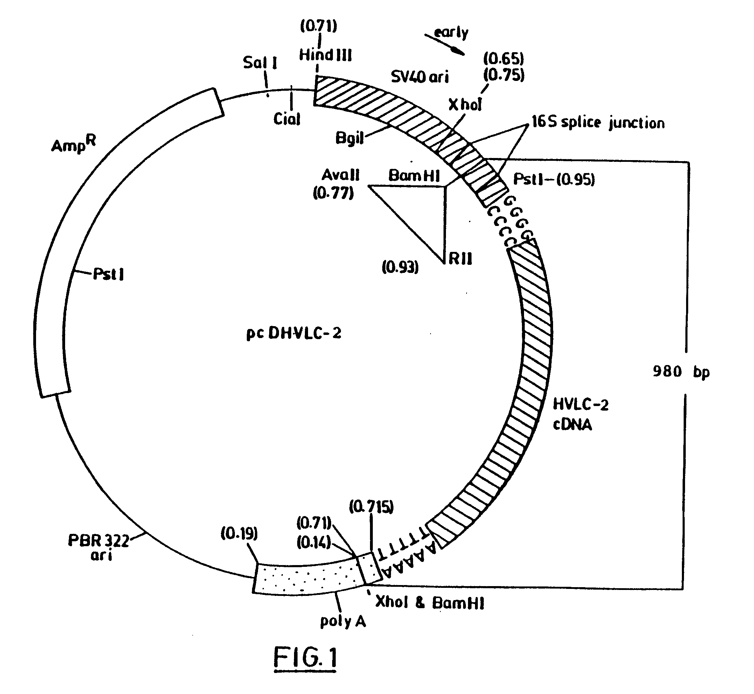

The total RNA was separated by the guanidine thiocyanate/cesium chloride method from human ventricle. Poly A messenger RNA was isolated from the total RNA by oligo dT column chromatography. A human ventricular cDNA library was prepared by using the messenger RNA as a template. The cDNA library is deposited at the ATCC under the deposit number ATCC 67789. The cDNA library was then screened using a probe consisting of a DNA that encodes a portion of the amino acid sequence of HVMLC2 which was partially elucidated by Klotz et al, supra. A DNA segment containing a DNA sequence coding for HVMLC2 protein was contained in a resulting clone (pCD-HVLC2). The restriction map of pCD-HVLC2 is set forth in Figure 1. The plasmid pCD-HVLC2 is deposited at the ATCC under the deposit number ATCC 67791.

The DNA segment of the present invention having a sequence which codes for HVMLC2 protein or a fragment of the DNA segment can be incorporated in a known manner in a plasmid vector which ensures good expression of the HVMLC2 protein or a part of the protein. Examples of plasmid vectors which can be used are pBR322, pBR325, pUC7, pUC8, pUC18 and pUC19, or other generally accessible plasmids.

A number of unique restriction sequences for restriction enzymes are incorporated in the DNA segment identified in Table II and these provide access to nucleotide sequences which code for polypeptides that are unique to HVMLC2. It is possible to make use of the following recognition sites for restriction enzymes to prepare fragments of the DNA segment of the embodiment of the invention:

AsuI (G↓GNCC, positions 147 to 180, 182 to 254, 255 to 366, 367 to 422, and 423 to 645)
Fnu4HI (GC↓NGC, positions 147 to 306, 307 to 561, and 562 to 645)
DdeI (C↓TNAG, positions 147 to 154, 155 to 291, 292 to 427, and 428 to 645)
MnII (CCTC, positions 147 to 237, 238 to 357, 358 to 389, 390 to 429, 430 to 527, 528 to 540, 541 to 543 and 544 to 645)

ScrFI (CCNGG, positions 147 to 175, 176 to 224, 225 to 363, 364 to 605, and 606 to 645)

DNA fragments having sequences unique to HVMLC2 can also be constructed by chemical synthesis and enzymatic ligation reactions carried out in a manner known per se.

The HVMLC2 protein or a part of the protein can be obtained by expression in a suitable host cell in a manner known per se. Suitable host cells are procaryotic organisms, preferably E, coli, B. subtilis, S. lividans and mammalian and yeast cells, preferably Saccharomyces cerevisiae.

The DNA segment of the present invention allows those skilled in the art to prepare antibodies against HVMLC2 protein or a part of the protein which can be used in in vivo and in vitro analytical methods. Antibody against HVMLC2 protein or a part of the protein can be prepared by conventional procedures by injecting HVMLC2 protein or a part of the protein obtained according to the process of the invention, or synthetically synthesized HVMLC2 protein or a part of the protein, into a suitable host animal; then bleeding the animal and concentrating the antiserum. Monoclonal antibodies specific for HVMLC2 protein or a part of the protein can also be prepared using the known cell fusion method. As to details relating to the preparation of monoclonal antibodies reference can be made to Goding, J.W., Monoclonal Antibodies: Principles and Practice, 2nd Ed., Academic Press, London, 1986. It is possible according to the invention to use the antibodies against HVMLC2 protein or a part of the protein, particularly a part having a unique amino acid sequence to HVMLC2, as a reagent for detecting HVMLC2, for example in an ELISA, radioimmunoassy, or histochemical tests.

Polyclonal or monoclonal antibodies against the HVMLC2 protein or a part of the protein can be used as medicaments, in particular as medicaments for the prophylaxis and treatment of heart disease. The polyclonal or monoclonal antibodies may be administered alone, in mixtures, in combination with one or more pharmaceutically acceptable excipients, diluents and auxiliaries or they may be conjugated to a therapeutic agent using known methods. Suitable therapeutic agents are enzymes or free radical scavengers, for example superoxide dismutase, catalase and vitamin E. The polyclonal or monoclonal antibodies against the HVMLC2 protein may also be conjugated to a labelling agent and used to detect damaged cardiac tissue. The labelling agent may be various enzymes, fluorescent materials, luminescent materials and radioactive materials. Examples of suitable enzymes include horseradish peroxidase, alkaline phosphatase, or $\beta$-galactosidase, acetylcholinesterase; examples of suitable fluorescent materials include umbelliferone, fluorescein, fluorescein isothiocyanate, rhodamine, dichlorotriazinylamine fluorescein, dansyl chloride or phycoerythrin; examples of luminescent materials include luminol; and examples of suitable radioactive materials include radioactive iodine $I^{125}$, $I^{131}$ or tritium. The enzymes, fluorescent materials, luminescent materials and radioactive materials can be conjugated to the polyclonal or monoclonal antibodies using conventional methods known in the art.

The DNA segment of the present invention also allows those skilled in the art to select DNA fragments from the DNA sequence shown in Table I, and complementary mRNA fragments which are unique to HVMLC2/.The invention would also permit one skilled in the art to select oligonucleotide sequences from the known sequences of myosin light chains from atrial, thymus or skeletal muscle tissue which are unique to the myosin light chains of those tissues. In addition polypeptides unique to myosin light chain proteins of specific tissues can be deduced from the unique DNA sequences. The selected DNA and mRNA oligonucleotide fragments can be used as reagents to detect the presence or absence of a complementary oligonucleotide in a biological sample. In particular oligonucleotides identified from the DNA segment of the present invention can be used as probes in diagnostic tests for heart disease to detect the presence or absence of complementary oligonucleotides, such as DNA or mRNA nucleotide fragments, in a biological sample. Antibodies against the selected polypeptides can be prepared using known methods and these antibodies can be used in in vivo or in vitro analytical methods or as medicaments.

The selection of oligonucleotide fragments or polypeptides useful as specific reagents, or in preparing specific reagents to detect HVMLC2 in a biological sample or to use as medicaments, is facilitated by knowing the conformation of the HVMLC2 protein. For example, polypeptides located in the exposed sites of the HVMLC2 protein, as shown in Figure 2, would be particularly useful in preparing antibodies to be used in analytical methods or as medicaments.

The following examples below illustrate the invention.

EXAMPLE 1

Isolation of Total RNA From Human Ventricle Using Guanidine Thiocynate

2g of human ventricle frozen in liquid $N_2$ was pulverized and homogenized in 20ml GTC solution (4M guanidine thiocynate, 25mM sodium citrate, 0.1M 2-mercaptoethanol, 0.5% sarkozyl) with a polytron (Brinkman Inst., PST 20 probe) at 5.5 rpm for 10 seconds, then homogenized in a teflon - glass homogenizer 2X low speed, the spun down in COREX tubes at 7,000 rpm for 10 minutes. Eight grams of cesium chloride were added to the supernatant and dissolved by vortexing. The solution was applied on 1.2 ml of cesium chloride solution (5.7 M cesium chloride, 25mM sodium acetate) and centrifuged at 35,000 rpm using a SW 50.1 rotor (6 tubes) for 16 hours at 20°C.

The GTC solution was removed, the sample was rinsed with fresh GTC solution and the cesium chloride was poured out. The tubes were cut 1.5 cm from the bottom. The RNA pellet (transparent) was dissolved in 500 $\mu$l of autoclaved $H_2O$, immediately transferred into 100% ETOH + 10% of 3M sodium acetate, pH 5.2, and left on ice for 16 hours at -20°C to precipitate. The amount of RNA was estimated by reading O.D.$_{260}$ - 330 $\mu$g. Several preparations were performed.

EXAMPLE 2

Isolation of Poly (A)$^+$ mRNA From Total RNA Using Oligo dT Column

100 mg oligo dT cellulose (Pharmacia) was resuspended in loading buffer (20 mM TrisHCl pH 7.6, 0.5M NaCl, 1mM EDTA, 0.1% SDS). The mixture was poured into a column (BIORAD, 2 ml disposable poly-prep chromatography column) and was washed with 2ml sterile $H_2O$, 2 ml 0.1M NaOH + 5 mM EDTA, 5 ml sterile $H_2O$). The pH of the eluent was 6.8. The column was washed with 5 column volumes of loading buffer. 500 $\mu$g of total RNA (combined preparations) were used. O.D.$_{260}$ was checked to confirm the amount. RNA was dissolved in 500 $\mu$l $H_2O$ (autoclaved) and heated up to 65°C for 5 minutes. An equal amount of 2 X loading buffer was added. The sample was cooled down to room temperature and applied on the column. The flow through was collected and heated again to 65°C and cooled down to room temperature. The sample was reapplied on the column. The column was washed with 10 column volumes of loading buffer and 4 column volumes of 0.1 M NaCl loading buffer. Poly A$^+$ was eluted with 3 column volumes of elution buffer (10mM Tris-HCl pH 7.5, 1 mM EDTA, 0.05% SDS). The Poly A$^+$ fraction was immediately precipitated with 100% ETOH + 10% of 3 M sodium acetate pH 5.2 and stored in -20°C. The amount of Poly A$^+$ RNA was estimated by reading O.D.$_{260}$ - 20 $\mu$g. The yield was approximately 4.2%.

EXAMPLE 3

Preparation of the Human Ventricular cDNA Library

The Okayama - Berg procedure (Mol. & Cell. Biol. 2, p. 161,. 1982) as set out in the cDNA cloning Manual by Pharmacia was used to make the cDNA library. All reagents were bought from Pharmacia. The method used to make the cDNA library is briefly described below.

The mRNA was tested using oligo p(dT)$_{12-18}$-primed reverse transcription to determine the size range of the mRNA population and its ability to serve as a template for reverse transcription. Poly A$^+$ mRNA (10 $\mu$g) was hybridized to oligo (dT) tailed pCDV-1 expression vector and was reverse transcribed using AMV reverse transcriptase. The cDNA was synthesized directly onto the vector, thus requiring no insertion steps. An oligo (dC)-tail (7 dC residues per 3' end) was added to the 3'-terminus of both the cDNA and the vector using terminal transferase. The dC-tail was removed from the 3' end of the vector using Hind III digestion. The oligo (dC)-tail Hind III cleaved cDNA:RNA vector was then circularized using oligo (dG)-tailed linked DNA (pL-1, 524 bp, 1/10 scale reaction). The RNA strand in the cDNA:RNA plasmid was replaced with DNA through nick translation using RNaseH, DNA polymerase I and DNA ligase (1/10 scale). The circular cDNA vector was then transformed into E. coli k12 strain MC1061 (1/10 scale) to create a cDNA library. Once the conditions were determined the procedure was increased 10 fold to obtain the cDNA library.

The cDNA library was stored at -70°C in 1.5 ml aliquots which were quick frozen in liquid nitrogen. The cDNA:RNA plasmid transformed into E coli K12 strain MC1061 is deposited at ATCC under the deposit number ATCC 67789.


EXAMPLE 4


Design and Construction of the Synthetic Oligonucleotide Probe


The synthetic oligonucleotide probe shown in Table V was derived from the partial amino acid sequence determined from chymotryptic peptides of human cardiac light chain 2 (HVMLC2) protein published by Klotz et al (Cir. Res. 50, p. 201, 1982). The probe was derived from the amino terminal end of the HVMLC2 protein corresponding to positions 27 - 32 of HVMLC2. The detailed amino acid sequence of HCMLC2 was not known. However, the same amino acid sequence for the synthetic oligonucleotide probe (Gln-GLu-Phe-Lys-Glu-Ala) is found in rabbit cardiac muscle light chain LC2 (Collins et al, supra) and rat cardiac light chain LC2 (Kumar et al, supra). This suggested that the amino acid sequence for the synthetic probe is well conserved in the genes of different muscles and different animals. Therefore, this probe was used to screen the human ventricular cDNA library. The synthetic oligonucleotide probe was synthesized by an automatic DNA-synthesizer (Pharmacia Gene Assembler, using cyanoethyl phosphoramidite).

Table V

| 17-mer probe for HVCMLC-2 32 combinations | | | | | |
|---|---|---|---|---|---|
| A | A | T | A | A | |
| CAG Gln | GAG Glu | TTC Phe | AAG Lys | GAG Gly | GC- Ala |


EXAMPLE 5


Purification of the Synthetic Oligonucleotide Probe


Cassettes containing the synthetic oligomer were spun down in Eppendorf tubes, dried on speed-vac and soaked in 25% NH₄OH (1.2 ml). The Eppendorf tubes were sealed with parafilm and soaked in 25% NH₄OH to minimize the leakage and left for 16 hours at 50°C. After cooling down on ice, the cassettes were transferred into new Eppendorf tubes and spun down to get all the liquid out. Both supernatants were combined and divided into 4 tubes and dried using the speed vac. The pellets were dissolved in 60 $\mu$l of $H_2O$. 30 $\mu$l DNA sample buffer (0.4% bromophenol blue, 25% ficol, 100 mM EDTA) was added, the solution was left for 5 minutes at 65°C, and then run on 20% acrylamide-urea gel (26.6 ml of 45% acrylamide-bis; 1.5% of 43% acrylamide-bis; 25.2g of Urea; 10X TBE (6.0 ml 0.89 M Tris-borate, 0.89 M boric acid, 0.02M -EDTA); 6.0 ml of $H_2O$; 15 mg of APS (ammonium persulfate); and, 40 $\mu$l TEMED).

The gel was run for approximately 3 hours at a constant current of 20mA, using 0.089M Tris-borate, 0.089M boric acid and 0.002M EDTA as a running buffer. The gel was stained with Ethidium Bromide (0.2 mg/ml in $H_2O$) for 15 minutes. Under UV light, the band corresponding to the size of the 17 mer was cut out, pulverized and resuspended in 8 ml of 0.5 M NH₄OAc, left at 37°C for 16 hours and then, spun down. The supernatant was extracted with $H_2O$ saturated butanol, and the oligomer was precipitated with 5 vol. of 100% ETOH + 10% 3 M sodium acetate pH 5.2. The oligomer was put on dry ice for 1 hour. The pellet was collected by centrifugation in a microfuge for 15 minutes and washed with 70% ETOH. The final pellet containing the purified oligomer was dried using a speed - vac and stored at 4° C. The purified oligomer was dissolved in 50 μl H₂O and the concentration was determined by A₂₆₀ nm (2.5 μg/μl). Approximately 200 ng of oligomer was used for 5' end labelling.


EXAMPLE 6


Screening of the cDNA Library Using Oligonucleotide Probes


An aliquot of the cDNA library was thawed on ice, centrifuged at 1200 rpm for 2 minutes, the pellet was dissolved in 1 ml LB (Luria Bertani medium - 10g Tryptone, 5 g yeast extract, 5 g sodium chloride, pH 7.5) and put into 10ml LB + ampicillin (50 μg/ml). The culture was grown for 6 hours at 37° C, titred and plated the next day. The E. coli transformants were grown on 40 small plates with approximately 5000 colonies per plate.

Nitrocellulose filters were placed on the culture plate and then put onto wet Whatman 3MM paper presoaked with 0.5m NaOH, 1M NaCl for 10 minutes, and dry blotted on Whatman 3MM paper. The filter was then transferred onto Whatman paper presoaked with 1M Tris, pH 7.4, 2.5 M NaCl for 10 minutes and dry blotted on Whatman's 3MM paper and the procedure was repeated again with 1M Tris, pH 7.4, 2.5M NaCl. The filters were air dried, baked in a vacuum oven at 80° C for 2 hours and wet in 3X SCC (20xSCC - 175.3g NaCl 88.2g sodium citrate). The filter was put in prehybridization buffer (6X SSC, 1X Denhardt's solution, 0.5% SDS, 100 μg/ml sheared and denatured salmon sperm DNA, 0.05% sodium pyrophosphate) and incubated for at least 2 hours at 37° C. The prehybridization buffer was removed and replaced with hybridization buffer (6X SSC, 1X Denhardt's 20 μg/ml tRNA, 0.05% sodium pyrophosphate) and 5' end labelled oligonucleotide probe. The purified synthetic oligonucleotide probe (example 5) was labelled by mixing 2 μl + 17-mer (0.2 μg), 27 μl H₂O, 10 μl 5x forward reaction buffer (5' DNA Terminus Labelling System from BRL) 9 μl ³²ATP SA 5000 Ci/mM, and 2 μl T-4 Kinase (10U/ml) and incubating for one hour at 37° C. Hybridization was carried out at 37° C for 16 hours. The nitrocellulose filters after the hybridization were washed in 6X SCC + 0.05% sodium pyrophosphate. The filters were washed twice at 37° C for 1/2 hour each and once at 47° C for 10-15 minutes. The filters were briefly air dried. Autoradiography was performed using an x-ray film (Kodak X-Omat RP) for 16 hours at -70° C.

Positives were obtained in primary screening, of which two proved to be true in secondary screening. One pCD-HVLC2 clone contained the complete 3' untranslated and coding regions. Figure 1 shows a schematic illustration of the restriction map of pCD-HVLC2. The plasmid pCD-HVLC2 is deposited at ATCC under the deposit number ATCC 67791.


EXAMPLE 7


Northern blot analysis was performed with a BamHI restriction fragment from plasmid pCD-HVLC2 according to the procedure of "B.D. Hames & S.J. Higgins, "Nucleic Acid Hybridization", A Practical Approach, IRL Press, 1985. Human atrial total mRNA as a negative control and human ventricular total mRNA were separated by electrophoresis, transferred to a nitrocellulose filter and hybridization was carried out with ³²P labelled BamHI restriction fragments from plasmid pCD-HVLC2. A transcript of approximately 1500 bp which corresponds to the size of HVMLC2 mRNA was detected with the human ventricular mRNA.


EXAMPLE 8


Southern blot analysis of DNA restriction fragments from the plasmid pCD-HVLC2 was performed according to the procedure of B.D. Hames & S.J. Higgins, "Nucleic Acid Hybridization A Practical Approach", IRL Press, 1985. Sall, Bam HI and Pst I restriction fragments from the plasmid pCD-HVLC2 were separated by gel electrophoresis and then transferred to a nitrocellulose filter. The immobilized DNA fragments were then probed with the labelled 17-mer oligonucleotide probe (Example 6). The Southern Blot analysis of the DNA restriction fragments from the plasmid pCD-HVLC2 probed with the 17 mer oligonucleotide probe demonstrated that the insert contained HVMLC2 specific sequences.

EXAMPLE 9

Determination of the Base Sequence of HVMLC2 cDNA

The HVMLC2 cDNA was sequenced using the dideoxy chain determination method. (Biggin, M. et al. (1983) Proc. Natl. Acad. Sci. U.S.A. 80, p. 3963-3965). The amino acid sequence deduced from the cDNA was compared with the known partial amino acid sequence of human cardiac myosin light chain 2 as described in the literature (Klotz, Circulation Research, 50, p. 201, 1982).

TABLE I

HVLC2

ATG GCA CCT AAG AAA GCA AAG AAG AGA GCC GGG GGC GCC AAC TCC AAC GTG TTC TCC ATG
Met Ala Pro Lys Lys Ala Lys Lys Arg Ala Gly Gly Ala Asn Ser Asn Val Phe Ser Met

TTC GAA CAG ACC CAA ATC CAG GAA TTT AAG GAG GCC TTC ACT ATC ATG GAC CAG AAC AGG
Phe Glu Gln Thr Gln Ile Gln Glu Phe Lys Glu Ala Phe Thr Ile Met Asp Gln Asn Arg

GAT GGC TTC ATT GAC AAG AAC GAT CTG AGA GAC ACC TTT GCT GCC CTT CGA GTG AAC GTG
Asp Gly Phe Ile Asp Lys Asn Asp Leu Arg Asp Thr Phe Ala Ala Leu Arg Val Asn Val

AAA AAT GAA GAA ATT GAT GAA ATG ATC AAG GAG GCT CCG GGT CCA ATT AAC TTT ACT GTG
Lys Asn Glu Glu Ile Asp Glu Met Ile Lys Glu Ala Pro Gly Pro Ile Asn Phe Thr Val

TTC CTC ACA ATG TTT GGG GAG AAA CTT AAG GGA GCG GAC CCT GAG GAA ACC ATT CTC AAC
Phe Leu Thr Met Phe Gly Glu Lys Leu Lys Gly Ala Asp Pro Glu Glu Thr Ile Leu Asn

GCA TTC AAA GTG TTT GAC CCT GAA GGC AAA GGG GTG CTG AAG GCT GAT TAC GTT CGG GAA
Ala Phe Lys Val Phe Asp Pro Glu Gly Lys Gly Val Leu Lys Ala Asp Tyr Val Arg Glu

ATG CTG ACC ACG CAG GCG GAG AGG TTT TCC AAG GAG GAG GTT GAC CAG ATG TTC GCC GCC
Met Leu Thr Thr Gln Ala Glu Arg Phe Ser Lys Glu Glu Val Asp Gln Met Phe Ala Ala

TTC CCC CCT GAC GTG ACT GGC AAC TTG GAC TAC AAG AAC CTG GTG CAC ATC ATC ACC CAC
Phe Pro Pro Asp Val Thr Gly Asn Leu Asp Tyr Lys Asn Leu Val His Ile Ile Thr His

GGA GAA GAG AAG GAC TAG
Gly Glu Glu Lys Asp

EP 0 357 856 A1

TABLE II

```
                       10         20         30         40         50
    First nt.           |          |          |          |          |
        +1      TGGTGGTGCA AATCAAAGAA CTGCTCCTCA GTGGATGTTG CCTTTACTTC ...

        +101    GGAGTGTGGA ATTCTTCTCG GGAGGCAGTG CTGGGTCCTT TCCACCATGG

        +201    TCCATGTTCG AACAGACCCA AATCCAGGAA TTTAAGGAGG CCTTCACTAT

        +301    CCTTTGCTGC CCTTCGAGTG AACGTGAAAA ATGAAGAAAT TGATGAAATG

        +401    TGGGGAGAAA CTTAAGGGAG CGGACCCTGA GGAAACCATT CTCAACGCAT

        +501    CGGGAAATGC TGACCACGCA GGCGGAGAGG TTTTCCAAGG AGGAGGTTGA

        +601    AGAACCTGGT GCACATCATC ACCCACGGAG AAGAGAAGGA CTAGGAGGGG

        +701    TCTCTCCCCC GAGTACCGCC TCTGTCCC
```

```
                       60         70         80         90         100
                        |          |          |          |          |
        ... TAGGCCTGTA CGGAAGTGTT ACTTCTGCTC TAAAAGCTGC TGCAGGGGGG

            CACCTAAGAA AGCAAAGAAG AGAGCCGGGG GCGCCAACTC CAACGTGTTC

            CATGGACCAG AACAGGGATG GCTTCATTGA CAAGAACGAT CTGAGAGACA

            ATCAAGGAGG CTCCGGGTCC AATTAACTTT ACTGTGTTCC TCACAATGTT

            TCAAAGTGTT TGACCCTGAA GGCAAAGGGG TGCTGAAGGC TGATTACGTT

            CCAGATGTTC GCCGCCTTCC CCCCTGACGT GACTGGCAAC TTGGACTACA

            GCTCGCTGCT GGCCCCTAAA CTCGTCTTTG CAGAGTGGTC CTGCCTCATC
```

## Table III

met-ala-pro-lys-lys-als-lys-lys-arg-<u>ala</u>-gly-<u>gly-ala</u>-asn-ser-<u>asn-val-phe</u>-ser-<u>met</u>-

<u>phe-glu-gln</u>-thr-<u>gln-ile-gln-glu-phe-lys-glu-ala-phe</u>-thr-ile-<u>met-asp-gln-asn-arg</u>-

<u>asp-gly-phe-ile-asp-lys-asn-asp-leu-arg-asp-thr-phe-ala-ala</u>-leu-arg-val-asn-val-

lys-asn-glu-glu-ile-asp-glu-met-ile-lys-glu-ala-pro-gly-pro-ile-asn-phe-thr-val-

phe-leu-thr-<u>met-phe-gly-glu-lys-leu-lys</u>-gly-ala-asp-pro-glu-glu-thr-ile-leu-asn-

<u>ala-phe-lys-val-phe-asp</u>-pro-glu-<u>gly-lys-gly</u>-val-<u>leu</u>-lys-<u>ala-asp-tyr-val-arg</u>-glu-

met-<u>leu-thr-thr-gln-ala-glu</u>-arg-<u>phe</u>-ser-lys-<u>glu-glu</u>-val-<u>asp-gln-met-phe-ala-ala</u>-

<u>phe-pro-pro-asp-val-thr-gly-asn-leu-asp-tyr-lys-asn</u>-leu-val-his-<u>ile-ile-thr-his</u>-

<u>gly-glu</u>-glu-<u>lys</u>-asp-STOP

Comparison of the deduced amino acid sequence of the HVMLC2 protein encoded in the DNA segment of one embodiment of the invention with the partial amino acid sequence disclosed by Klotz et al. The lines underline the residues corresponding to the part of the amino acid sequence disclosed by Klotz et al.

Table IV

# COMPARISON OF VENTRICULAR MYOSIN LIGHT CHAIN 2 SEQUENCE IN MAMMALS

```
Human   A P K K A K K R A G G A N S N V F S M F E Q T Q I Q E F K E A
Rabbit              "  Ξ "  "  " "  "    "  "  "  "  " "  "  "  "  "  "  " "  "  "  "  "
Rat     "  "  "  "  "  "  "  L.E "  G  S"  "   " "  "  "  "  " "  "  "  " "  "  "  "  "

Human   F.T I M D Q N R A G F I D K N D L R D T F A A L - R V N V K
Rabbit  "  "  "  "  "  "  "  "  " "  "  "  "  "  " "  "  "  "  "  "  "  G "  "  "  "  "
Rat     "  "  "  "  "  "  "  "  " "  "  "  "  "  "  "  " "  "  "  "  "  "  "  "  "  "

Human   N E E I D E M I K E A P G P I N F T V F L T M F G E K L K G
Rabbit  "  "  " "  "  "  "  " "  "  "  "  "  "  "  " "  "  "  "  "  " "  "  "  "
Rat     "  "  " "  "  "  "  "  " "  "  "  "  "  "  "  " "  "  "  "  "  R " "  "  "  "

Human   A D P E E T L L N A F K V F D P.E G K G V L K A D Y V R E
Rabbit  "  "  "  " "  "  " "  "  "  "  " "  "  "  " "  "  "  " "  "  " "  "  "  " "  "
Rat     "  G  " " "  " " "  "  "  " " "  "  "  A " R R R I  T G - "  " L Y Q "

Human   M L T T Q E R F S K E E V D Q M F A A F P P D V T G N L D Y
Rabbit  "  "  " " "  " "  " "  "  " D " I"  "  "  "  " "  "  "  "  " "  "  "  "  " "  "
Rat     "  "  " " "  " "  " "  "  " E " T "  "  "  " "  "  "  "  "  N "  " S "  "  "  "  "

Human   K N L V H I I T H G E E K D
Rabbit  "  "  " " "  " "  " "  "  "  " "  " I
Rat     "  "  " " "  " "  " "  " "  "  "  " "
```

| | | | | | |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamic acid | Glu | E | Serine | Ser | S |
| Glutamine | Gln | Q | Threonine | Thr | T |
| Glycine | Gly | G | Tryptophan | Trp | W |
| Histidine | His | H | Tyrosine | Tyr | Y |
| Isoleucine | Ile | I | Valine | Val | V |

**Claims**

1. A DNA segment having a sequence which codes for human ventricular myosin light chain 2 protein

EP 0 357 856 A1

or a fragment of the DNA segment.

2. A DNA segment having a sequence which codes for human ventricular myosin light chain 2 protein of the amino acid sequence as shown in Table I or a fragment of the DNA segment.

3. A DNA segment having a sequence which codes for human ventricular myosin light chain 2 protein of the nucleotide sequence as shown in Table I or a fragment of the DNA segment.

4. The DNA segment as claimed in claim 1, which has the nucleotide sequence as shown in Table II.

5. A recombinant plasmid adapted for transformation of a host cell comprising a plasmid vector into which a DNA segment or a fragment of a DNA segment as claimed in any one of claims 1 to 4 has been inserted.

6. The recombinant plasmid as claimed in claim 5, wherein the plasmid vector comprises E. coli plasmid pBR322.

7. The recombinant plasmid as claimed in claims 5 or 6, which includes Pst I restriction endonuclease sites.

8. The recombinant plasmid as claimed in claims 5 or 6, which includes Bam HI restriction endonuclease sites.

9. The recombinant plasmid as shown in Figure 1.

10. A transformant host plasmid as claimed in any one of claims 5 to 9.

11. The transformed host cell as claimed in claim 10, wherein the host cell is a bacterial cell.

12. The transformant host cell as claimed in claims 10 or 11 wherein the host cell is E. coli.

13. A process for producing human ventricular myosin light chain 2 protein or a part of the protein comprising culturing a transformant host cell as claimed in claims 10, 11 or 12, in a suitable medium until human ventricular myosin light chain 2 protein or part of the protein is formed and isolating the human ventricular myosin light chain 2 protein or part of the protein.

14. A polyclonal antibody directed against human ventricular myosin light chain 2 protein or a part of the protein, which is prepared in a manner known per se using the human ventricular myosin light chain 2 protein or a part of the protein prepared according to the process as claimed in claim 13 or synthetically synthesized HVMLC2 protein or a part of the protein.

15. A monoclonal antibody directed against human ventricular myosin light chain 2 protein or a part of the protein, which is prepared in a manner known per se using the human ventricular myosin light chain 2 protein or a part of the protein prepared according to the process as claimed in claim 13 or synthetically synthesized HVMLC2 protein or a part of the protein.

16. A medicament for the prophylaxis and treatment of heart disease which comprises a polyclonal antibody as claimed in claim 14 and one or more of a therapeutic agent and a pharmaceutically acceptable excipient, diluent and auxiliary.

17. A medicament for the prophylaxis and treatment of heart disease which comprises a monoclonal antibody as claimed in claim 15 and one or more of a therapeutic agent and a pharmaceutically acceptable excipient, diluent and auxiliary.

18. An agent for detecting damaged cardiac tissue which comprises a polyclonal antibody as claimed in claim 14 and a labelling agent.

19. An agent for detecting damaged cardiac tissue which comprises a monoclonal antibody as claimed in claim 15 and a labelling agent.

20. The agent as claimed in claims 18 or 19, wherein the labelling agent is a radioactive material.

21. A process for producing a DNA segment according to any one of claims 1 to 4, a recombinant plasmid according to any one of claims 5 to 9, or a transformed host according to any one of claims 10 to 12.

22. Use of polyclonal antibody according to claim 14 or a monoclonal antibody according to claim 15 in the manufacture of a medicament for the prophylaxis and treatment of heart disease or in the manufacture of an agent for detecting damaged cardiac tissue.

14

EP 0 357 856 A1

FIG.1

HVMLC 2

NH$_2$ terminal

COOH terminal

FIG. 2

EP 0 357 856 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BIOLOGICAL ABSTRACTS, no. 32088807, 1987; C.C. KUMAR et al.: "Cloning and characterization of smooth muscle myosin light chain-2 from chicken and human tissues", & Biophysical Journal (US), 1987, vol. 51, no. 2 Part 2 p 120A * Abstract * --- | 1-13,21 | C 12 N 15/00<br>C 12 P 21/02<br>C 07 K 15/00<br>C 12 P 21/00<br>A 61 K 39/395<br>G 01 N 33/68 //<br>(C 12 P 21/00<br>C 12 R 1:91 ) |
| Y | CYTOGENET. CELL GENET, vol. 40, 1985, page 574; L. BALAZS et al.: "Chromosomal mapping and characterization of human DNA sequences homologous to cardfac myosin light chain gene" * Whole article * --- | 1-13,21 | |
| Y | THE JOURNAL OF BIOLOGICAL CHEMISTRY, vol. 257, no. 18, 25th September 1982, pages 11078-11086; L.I. GARFINKEL et al.: "Cloning and characterization of cDNA sequences corresponding to myosin light chains 1,2, and 3, Troponin-C, Troponin-T, alpha-Tropomyosin, and alpha-Actin" * Whole article * --- | 1-13,21 | TECHNICAL FIELDS SEARCHED (Int. Cl.4)<br><br>C 12 N<br>G 01 N<br>A 61 K |
| Y | NUCLEIC ACIDS RESEARCH, vol. 16, no. 10, 1988, page 4722, IRL Press Ltd, Oxford, GB; S.A. HENDERSON et al.: "Nucleotide sequence of full length cDNAs encoding rat cardiac myosin light chain-2" * Whole article * --- -/- | 1-13,21 | |

The present search report has been drawn up for all claims

| Place of search | Date of completion of the search | Examiner |
|---|---|---|
| THE HAGUE | 18-05-1989 | PULAZZINI A.F.R. |

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | THE JOURNAL OF BIOLOGICAL CHEMISTRY, vol. 261, no. 6, 25th February 1986, pages 2866-2872, The American Society of Biological Chemists, Inc., US; C. CHANDRA KUMART et al.: "Heart myosin light chain 2 gene. Nucleotide sequence of full length cDNA and expression in normal and hypertensive rat" * Whole article * | 1-13,21 | |
| X | EP-A-0 205 177  (YAMASA SHOYU K.K.) * Whole document * | 14-22 | |
| D,X | BIOCHEMICAL AND BIOPHYSICAL RESEARCH COMMUNICATIONS, vol. 86, no. 3, 14th February 1979, pages 683-688, Academic Press, Inc.; R. NAGAI et al.: "Radioimmunoassay of cardiac myosin light chain II in the serum following experimental myocardial infarction" * Whole article * | 14,18, 20 | |
| | | | TECHNICAL FIELDS SEARCHED (Int. Cl.4) |
| X | CHEMICAL ABSTRACTS, vol. 103, no. 7, August 1985, page 216, abstract no. 49971b, Columbus, Ohio, US; T. SHIMIZU et al.: "Analysis of the metal-induced conformational change in myosin with a monoclonal antibody to light chain two", & J. MOL. BIOL. 1985, 183(2), 271-82 * Whole abstract *                -/- | 14 | |

The present search report has been drawn up for all claims

| Place of search | Date of completion of the search | Examiner |
|---|---|---|
| THE HAGUE | 18-05-1989 | PULAZZINI A.F.R. |

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | JOURNAL OF MOLECULAR AND CELLULAR CARDIOLOGY, vol. 14, suppl. 3, 1982, pages 139-146; E. HABER et al.: "Detection and quantification of myocardial cell death: Application of monoclonal antibodies specific for cardiac myosin" | | |

TECHNICAL FIELDS
SEARCHED (Int. Cl.4)

The present search report has been drawn up for all claims

| Place of search | Date of completion of the search | Examiner |
|---|---|---|
| THE HAGUE | 18-05-1989 | PULAZZINI A.F.R. |

EPO FORM 1503 03.82 (P0401)